# FASCICULE DE BREVET EUROPEEN

(11) **EP 1 644 526 B1**
(45) Date de publication et mention de la délivrance du brevet: **07.05.2008**
(21) Numéro de dépôt: 04767876.8
(22) Date de dépôt: 08.07.2004
(51) Int. Cl.: C12Q 1/68

(54) **PROCEDE DE DETECTION ET/OU D IDENTIFICATION DE BACTERIES DE GENRE STAPHYLOCOCCUS**
VERFAHREN ZUM NACHWEIS UND/ODER ZUR IDENTIFIZIERUNG VON BAKTERIEN DES GENUS STAPHYLOCOCCUS
METHOD FOR DETECTING AND/OR IDENTIFYING BACTERIA OF GENUS STAPHYLOCOCCUS

(30) Priorité: 10.07.2003 FR 0308446
(43) Date de publication de la demande: 12.04.2006
(73) Titulaire: BIOMERIEUX, 69280 Marcy-L'Etoile (FR)
(72) Inventeur: TROESCH, Alain, F-69740 Genas (FR); LACROIX, Bruno, F-69230 Saint-Genis Laval (FR); JAY, Corinne, F-69300 Caluire (FR)
(74) Mandataire: Denjean, Frédérique
(86) Numéro de dépôt international: PCT/FR2004/050315
(87) Numéro de publication internationale: WO 2005/007883

(56) Documents cités:
- WO-A-00/66788
- WO-A-02/08265
- JP-A- 6 090 798
- JP-A- 2001 095 579
- JP-A- 2002 051 783
- US-A- 5 714 321
- DATABASE EMBL [en ligne] 15 juin 1993 (1993-06-15) "S.aureus gene for 16S rRNA" retrieved from EBI, accession no. EM_PRO:SA16SRRN Database accession no. X68417 XP002317395
- SCHMITZ F -J ET AL: "Specific information concerning taxonomy, pathogenicity and methicillin resistance of staphylococci obtained by a multiplex PCR" JOURNAL OF MEDICAL MICROBIOLOGY, vol. 46, no. 9, 1997, pages 773-778, XP008028561 ISSN: 0022-2615
- EDWARDS K J ET AL: "Rapid and accurate identification of coagulase-negative staphylococci by real-time PCR" JOURNAL OF CLINICAL MICROBIOLOGY, WASHINGTON, DC, US, vol. 39, no. 9, septembre 2001 (2001-09), pages 3047-3051, XP002242459 ISSN: 0095-1137
- MASON W J ET AL: "Multiplex PCR protocol for the diagnosis of staphylococcal infection" JOURNAL OF CLINICAL MICROBIOLOGY 2001 UNITED STATES, vol. 39, no. 9, 2001, pages 3332-3338, XP002272884 ISSN: 0095-1137

### LISTAGE DES SEQUENCES

<110> BIOMERIEUX SA
<120> Procédé de détection et/ou d'identification de bactéries du genre Staphylococcus
<130> Unknown
<160> 2
<170> PatentIn version 3.1
<210> 1
   <211> 20
   <212> DNA
   <213> Staphylococcus sp.
<400> 1
   taacctacct ataagactgg 20
<210> 2
   <211> 20
   <212> DNA
   <213> Staphylococcus sp.
<400> 2
   tcaccccaat catttgtccc 20

## Description

La présente invention concerne la détection et/ou l'identification de bactéries de genre *Staphylococcus.*

Les bactéries de genre *Staphylococcus* ou Staphylocoques sont responsables d'un grand nombre d'infections nosocomiales et représentent un problème hospitalier important. Ces bactéries sont des bactéries Gram positives, qui peuvent être classées en deux grands groupes que l'on distingue par la production d'une enzyme, la coagulase, déclenchant la coagulation du plasma. On distingue ainsi les Staphylocoques coagulase négatifs, dont le représentant principal est *Staphylococcus epidermidis* et les Staphylocoques coagulase positifs, dont le représentant principal est *Staphylococcus aureus,* bien connu pour sa virulence. Les Staphylocoques sont largement répandus dans l'environnement, la peau et les muqueuses de l'homme et de l'animal. La desquamation régulière de ces hôtes a pour effet de les disperser abondamment dans la nature (eau, sol, air, aliments, objets), et en milieu hospitalier, des mesures draconiennes d'hygiène et d'isolement des patients sont requises pour limiter la dissémination des souches épidermiques. Ainsi, *Staphylococcus aureus,* qui représente 80 à 90% des Staphylocoques isolés en clinique est un pathogène majeur de l'homme responsable de nombreuses infections en milieu hospitalier, tels que notamment lors de pneumonies nosocomiales, d'infections de plaies chirurgicales, d'infections de brûlures, d'infections des corps étrangers (valves cardiaques, prothèses de hanche, agrafes...) et d'infections systémiques ou de septicémies qui sont souvent dues à l'usagé de cathéters intra vasculaires, ou à la dissémination de la bactérie à partir d'un autre foyer infectieux. D'autres Staphylocoques font naturellement partie de la flore cutanéo-muqueuse de l'homme comme *Staphylococcus epidermidis,* mais possèdent également un potentiel pathogène, qui s'exprime alors dans un contexte particulier: implantation de corps étrangers (prothèse cardiaque, sondes, cathéters...) et/ou immunodéficience (sida, radiothérapie, chimiothérapie, néonatalité...).

Il s'avère ainsi que n'importe quel Staphylocoque siégeant de façon permanente sur la peau et les muqueuses de l'homme présente un risque infectieux. Un diagnostic précoce de la présence de ces bactéries, ainsi que leur identification s'avère donc essentiel.

Un premier examen microscopique permet, rapidement, de part la morphologie des bactéries Gram positive très évocatrice, de suspecter la présence de Staphylocoques dans un échantillon biologique, tel qu'un échantillon sanguin ou un échantillon issu d'une plaie infectée.

Si cet examen microscopique montre la présence de bactéries Gram positive, un examen plus approfondi doit être réalisé, afin d'une part, de confirmer que ces bactéries appartiennent bien au genre *Staphylococcus,* et s'assurer qu'il ne s'agit pas de bactéries Gram positive d'autres genre telles que des bactéries de genre *Micrococcus* et d'autre part d'identifier l'espèce à laquelle appartiennent ces bactéries (*Staphylococcus epidermis, aureus*...). Pour cela, on met en culture ces bactéries (sur milieu standard et milieu sélectif de Chapman) et on réalise :
- un diagnostic du genre auquel appartiennent ces bactéries. Ce diagnostic de genre peut être réalisé par un test de sensibilité à la lysostaphine, au lysozyme ou à la nitrofurantoïne,
- un diagnostic de l'espèce à laquelle appartiennent ces bactéries qui peut être réalisé notamment par des galeries d'identification.

Les techniques de biologie moléculaire sont également utilisées pour le diagnostic de genre et d'espèce, et permettent d'établir un diagnostic rapidement. Le principe de fonctionnement de ces techniques repose principalement sur un des fondements de la biologie moléculaire : le phénomène d'hybridation (voir par exemple A.C. Whelen et D. Persing, Annu. Rev. Microbiol., 1996, 50, p 349-373 ; J. M. Hunt et D. H. Persing, « Bacteria detection » in DNA Probes, ed GH Keller and MM Macrak, Stockton Press, 2nd edition, Section 13, p525-55).

Le diagnostic de l'espèce et/ou de genre peut être déterminé par l'utilisation de sondes d'hybridation. Lorsqu'on compare le génome des différentes espèces de bactéries de genre *Staphylococcus,* on peut distinguer des régions dites conservées, dont la séquence est identique entre les différentes espèces de bactéries de genre *Staphylococcus* (on parlera par la suite de « séquence cible conservée »), et des régions dites spécifiques, dont la séquence est variable entre les différentes espèces de bactéries de genre *Staphylococcus* et caractéristique de chaque espèce (on parlera par la suite de « séquence cible spécifique »). Lorsqu'on souhaite réaliser un diagnostic d'espèce, on choisit une sonde d'hybridation, spécifique d'une espèce donnée, qui s'hybride par complémentarité à une séquence cible spécifique. Lorsqu'on souhaite réaliser un diagnostic de genre, on choisit une sonde d'hybridation spécifique de genre *Staphylococcus,* qui s'hybride, par complémentarité à une séquence cible conservée. Ces techniques de biologie moléculaire faisant appel à des sondes d'hybridation sont bien connues de l'homme du métier qui peut se référer notamment à un ouvrage de base tel que « Molecular cloning, a laboratory manual », voir notamment Chapitre 9: «analysis and cloning of eukaryotic genomic DNA » et paragraphe 9.47 : « Hybridization of radiolabeled probes to immobilized nucleic acids »; J Sambrook, EF Fritsch, T Maniatis, (eds Cold Spring Harbor Laboratory Press).

Toutefois, la quantité de bactéries étant généralement trop faible dans la plupart des prélèvements cliniques, il est souvent nécessaire d'augmenter, par une réaction d'amplification enzymatique, la quantité de séquences cibles (conservées ou spécifiques) susceptibles de s'hybrider à une sonde d'hybridation, afin d'augmenter la probabilité d'hybridation entre cette séquence cible et cette sonde d'hybridation. De telles techniques sont bien connues de l'homme du métier et on peut citer notamment la PCR (Polymerase Chain Reaction), telle que décrite dans les brevets US-A-4,683,195, US-A-4,683,202 et US-A-4,800,159.

Pour le diagnostic de bactéries de genre *Staphylococcus,* cette étape d'amplification est délicate car, ne connaissant ni l'espèce, ni le genre de bactéries susceptible de contaminer l'échantillon, on doit utiliser des amorces d'amplification permettant d'amplifier des séquences cibles uniquement de bactéries de genre *Staphylococcus,* mais ceci, quelque soit l'espèce de bactéries de genre *Staphylococcus.* De plus, du fait de la grande variabilité du génome entre les différentes espèces de bactéries de genre *Staphylococcus,* une amorce d'amplification conforme à la séquence connue d'une espèce de genre *Staphylococcus* peut faillir à l'amplification d'une autre espèce de genre *Staphylococcus.* Le choix des amorces d'amplification est donc essentiel.

Schmitz et al. (J. Med. Microbiol. (1997) 46: 773-778) décrit un procédé de détection ou d'identification de bactéries du genres Staphylococcus d'un échantillon biologique comprenant les étapes d'extraction du matériel nucléique des bactéries de genre Staphylococcus, l'amplification d'une séquence cible spécifique de bactéries du genre Staphylococcus grâce aux amorces d'amplification RDR327 et DG74 et la détermination de la présence de bactéries du genre Staphylococcus par la détection des amplicons.

La présente invention fournit précisément de nouvelles amorces d'amplification facilitant la détection et/ou l'identification d'un grand nombre d'espèce de bactéries de genre *Staphylococcus.* Lorsque qu'on ne connaît pas l'espèce de Staphylocoqques présente dans un échantillon, il est alors très pertinent d'utiliser des amorces selon l'invention, permettant d'amplifier toutes les espèces de genre *Staphylococcus.* On obtient alors une grande quantité de matériel biologique permettant d'utiliser ensuite une batterie de sondes spécifiques, pour déterminer, sans aucune présomption préalable, quelle espèce de Staphylocoques est présente dans l'échantillon.

D'une manière surprenante, les inventeurs ont découvert que les amorces d'amplification comprenant la SEQ ID N°1 (5' TAA CCT ACC TAT AAG ACTG G 3') ou la SEQ ID N°2 (5' TC ACC CCA ATC AIT TGT CCC 3') sont très pertinentes pour amplifier des séquences cibles permettant non seulement de détecter des bactéries de genre *Staphylococcus,* mais également d'identifier un grand nombre d'espèces de bactéries de genre *Staphylococcus.*

A ce titre, l'invention concerne un procédé de détection et/ou d'identification de bactéries de genre *Staphylococcus* dans un échantillon biologique comprenant les étapes suivantes :
A. on extrait le matériel nucléique des bactéries de genre *Staphylococcus,*
B. on amplifie au moins une séquence cible du matériel nucléique des bactéries de genre *Staphylococcus* grâce à au moins une amorce d'amplification de 15 à 25 motifs nucléotidiques comprenant au moins 10 motifs nucléotidiques de la SEQ ID N°1 et au moins une amorce d'amplification de 15 à 25 motifs nucléotidiques comprenant au moins 10 motifs nucléotidiques de la SEQ ID N°2 pour obtenir des amplicons de la séquence cible,
C. on détermine la présence de bactéries de genre *Staphylococcus* par la détection desdits amplicons.

Au sens de la présente invention, les bactéries de genre *Staphylococcus* peuvent être notamment des bactéries *Staphylococcus arlettae ; Staphylococcus aureus.aureus ; Staphylococcus auricularis ; Staphylococcus capitis.capitis ; Staphylococcus capitis.ureolyticus; Staphylococcus caprae ;Staphylococcus carnosus carnosus ; Staphylococcus carnosus utilis ; Staphylococcus chromogenes ; Staphylococcus cohnii cohnii ; Staphylococcus cohnii urealyticum ; Staphylococcus condimenti ; Staphylococcus delphini ; Staphylococcus epidermidis ; Staphylococcus equorum ; Staphylococcus gallinarum ; Staphylococcus haemolyticus ; Staphylococcus hominis.hominis ; Staphylococcus hominis.novobiosepticus ; Staplaylococcus hyicus ; Staphylococcus intermedius ; Staplzylococcus kloosii ; Staphylococcus lentus ; Staphylococcus lugdunensis ; Staphylococcus pasteuri ; Staphylococcus piscifermentans ; Staphylococcus pulvereri ; Staphylococcus saprophyticus.bovis ; Staphylococcus saprophyticus.saprophyticus ; Staphylococcus schleiferi.coagulans ; Staphylococcus schleiferi.schleiferi ; Staphylococcus sciuri ; Staphylococcus sinaulans ; Staphylococcus vitulinus ; Staphylococcus warneri ; Staphylococcus xylosus*

Au sens de la présente invention, on entend par échantillon biologique, tout échantillon susceptible de contenir un matériel nucléique tel que défini ci après. Cet échantillon biologique peut être prélevé chez un patient et peut être notamment un échantillon de tissus infectés, de prélèvements nasaux, de sang, de sérum, de salive, de cellules circulantes du patient. On dispose de cet échantillon biologique par tout type de prélèvement connu de l'homme du métier. Cet échantillon biologique peut également être un échantillon alimentaire, d'eau, de lait, aliment d'origine animale, prélèvements vétérinaires (tissus infectées...).

Au sens de la présente invention, le matériel nucléique comprend une séquence d'acides nucléiques telle que séquence d'acides désoxyribonucléiques (ADN) ou d'acides ribonucléiques (ARN). Par séquence d'acides nucléiques (ou fragment nucléotidique ou oligonucléotide, ou polynucléotide), on entend un enchaînement de motifs nucléotidiques assemblés entre eux par des liaisons ester phosphorique, caractérisé par la séquence informationnelle des acides nucléiques naturels, susceptibles de s'hybrider à une autre séquence d'acides nucléiques, l'enchaînement pouvant contenir des monomères de structures différentes et être obtenu à partir d'une molécule d'acide nucléique naturelle et/ou par recombinaison génétique et/ou par synthèse chimique. Par motif nucléique, on entend un dérivé d'un monomère qui peut être un nucléotide naturel d'acide nucléique dont les éléments constitutifs sont un sucre, un groupement phosphate et une base azotée; dans l'ADN le sucre est le désoxy-2-ribose, dans l'ARN le sucre est le ribose ; selon qu'il s'agisse de l'ADN ou l'ARN, la base azotée est choisie parmi l'adénine, la guanine, l'uracile, la cytosine, la thymine ; ou bien le monomère est un nucléotide modifié dans l'un au moins des trois éléments constitutifs ; à titre d'exemple, la modification peut intervenir soit au niveau des bases, avec des bases modifiées telles que l'inosine, la méthyl-5désoxycytidine, la désoxyuridine, la diméthylamino-5désoxyuridine, la diamino-2,6-purine, la bromo-sdésoxyulidine ou toute autre base modifiée capable d'hybridation, soit au niveau du sucre, par exemple le remplacement d'au moins un désoxyribose par un polyamide (P.E. Nielsen et al, Science, 254, 1497-1500 (1991), soit encore au niveau du groupement phosphate, par exemple son remplacement par des esters notamment choisis parmi les diphosphates, alkyl- et aryl-phosphonates et phosphorothioates.

Selon un mode préféré de réalisation de l'invention, le matériel nucléique comprend une séquence d'acides désoxyribonucléiques.

Cette séquence d'acides nucléiques comprend au moins une séquence cible. Par séquence cible, on entend une séquence dont l'enchaînement en motifs nucléotidiques permet de déterminer la présence d'une bactérie de genre *Staphylococcus* et/ou d'identifier l'espèce de bactérie de genre *Staphylococcus.* Cette séquence cible comprend au moins une séquence cible conservée, c'est à dire une séquence dont l'enchaînement en motifs nucléotidiques permet de déterminer la présence de n'importe quelle espèce de bactéries de genre *Staphylococcus.* La séquence cible peut comprendre, en outre, au moins une séquence cible spécifique, c'est à dire une séquence dont l'enchaînement en motifs nucléotidiques permet d'identifier une espèce donnée de bactérie de genre *Staphylococcus.* Selon un mode encore plus préféré de réalisation de l'invention, la séquence cible spécifique est comprise entre deux séquences cibles conservées.

Selon un mode préféré de réalisation de l'invention, le matériel nucléique est extrait d'un échantillon biologique prélevé chez un patient.

On entend par amorce d'amplification, une séquence nucléique comprenant de 5 à 100 motifs nucléotidiques, préférentiellement de 15 à 25 motifs nucléotidiques permettant l'initiation d'une polymérisation enzymatique, telle que notamment une réaction d'amplification enzymatique. Cette amorce d'amplification comprend au moins 10, préférentiellement 15 et encore plus préférentiellement 20 motifs nucléotidiques de la SEQ ID N°1 ou au moins 10, préférentiellement 15 et encore plus préférentiellement 20 motifs nucléotidiques de la SEQ ID N°2. Une amorce d'amplification comprenant
- une séquence homologue à la SEQ ID N°1 ou la SEQ ID N°2, c'est à dire
   o la séquence complémentaire ou suffisamment complémentaire de la SEQ ID N°1 ou 2
   o une séquence présentant une homologie suffisante pour s'hybrider à la SEQ ID N°1 ou à la SEQ ID °2 ou à la séquence complémentaire à la SEQ ID N°1 ou à la SEQ ID N°2,
- une séquence comprenant une séquence de SEQ ID N°1 ou SEQ ID N°2 (ou une séquence homologue à SEQ ID N°1 ou SEQ ID N°2 telle que définie précédemment) dans laquelle les bases uracile sont substituées aux bases thymine,
et qui aurait la même fonction que l'amorce d'amplification selon l'invention, c'est à dire permettre l'amplification d'un grand nombre d'espèces de bactéries de genre *Staphylococcus,* est également décrite.

Par réaction d'amplification enzymatique, on entend un processus générant de multiples copies (ou amplicons) d'une séquence nucléique par l'action d'au moins une enzyme. De telles réactions d'amplification sont bien connues de l'homme du métier et on peut citer notamment la PCR (Polymerase Chain Reaction), telle que décrite dans les brevets US-A-4,683,195, US-A-4,683,202 et US-A-4,800,159; la LCR (Ligase Chain Reaction), exposée par exemple dans la demande de brevet EP-A-0 201 184 ; la RCR (Repair Chain Reaction), décrite dans la demande de brevet WO-A-90/01069 ; la 3SR (Self Sustained Sequence Replication) avec la demande de brevet WO-A-90/06995 ; la NASBA (Nucleic Acid Sequence-Based Amplification) avec la demande de brevet WO-A-91/02818, ou encore la TMA (Transcription Mediated Amplification) avec le brevet US-A-5,399,491. Dans la suite de l'exposé, on parlera de séquence cible qu'elle soit en simple brin ou en double brin.

Lors de l'étape A, on extrait le matériel nucléique par tout protocole connu de l'homme du métier. A titre indicatif, l'extraction d'acides nucléique peut être réalisée par une étape de lyse des cellules présentes dans l'échantillon biologique, afin de libérer les acides nucléiques contenus dans les enveloppes protéiques et/ou lipidiques des microorganismes (comme des débris cellulaires qui perturbent les réactions ultérieures). A titre d'exemple, on peut utiliser les méthodes de lyse telles que décrite dans les demandes de brevet WO 00/05338 sur la lyse mixte magnétique et mécanique, WO 99/53304 sur la lyse électrique, et WO 99/15321 sur la lyse mécanique.

L'homme du métier pourra utiliser d'autres méthodes de lyse bien connues, telles que les chocs thermiques ou osmotiques ou les lyses chimiques par des agents chaotropiques tels que les sels de guanidium (US 5,234,809). Cette étape de lyse peut également être suivie d'une étape de purification, permettant la séparation entre les acides nucléiques et les autres constituants cellulaires relargués dans l'étape de lyse. Cette étape permet généralement de concentrer les acides nucléiques, et peut être adapté à la purification d'ADN ou d'ARN. A titre d'exemple, on peut utiliser des particules magnétiques éventuellement revêtues d'oligonucléotides, par adsorption ou covalence (voir à ce sujet les brevets US 4,672,040 et US 5,750,338), et ainsi purifier les acides nucléiques qui se sont fixés sur ces particules magnétiques, par une étape de lavage. Cette étape de purification des acides nucléiques est particulièrement intéressante si l'on souhaite amplifier ultérieurement lesdits acides nucléiques. Un mode de réalisation particulièrement intéressant de ces particules magnétiques est décrit dans les demandes de brevet WO97/45202 et WO99/35500. Un autre exemple intéressant de méthode de purification des acides nucléiques est l'utilisation de silice soit sous forme de colonne, soit sous forme de particules inertes (Boom R. et al., J. Clin. Microbiol., 1990, n°28(3), p. 495-503) ou magnétiques (Merck: MagPrep^{®} Silica, Promega: MagneSil^{™} Paramagnetic particles). D'autres méthodes très répandues reposent sur des résines échangeuses d'ions en colonne ou en format particulaire paramagnétique (Whatman: DEAE-Magarose) (Levison PR et al., J. Chromatography, 1998, p. 337-344). Une autre méthode très pertinente mais non exclusive pour l'invention est celle de l'adsorption sur support d'oxyde métallique (société Xtrana: matrice Xtra-Bind^{™}).

Lorsque l'on souhaite extraire spécifiquement l'ADN d'un échantillon biologique, on peut notamment réaliser une extraction par du phénol, du chloroforme et de l'alcool pour éliminer les protéines et précipiter l'ADN avec de l'alcool 100%. L'ADN peut alors être culoté par centrifugation, lavé et remis en suspension.

Lors de l'étape B, on amplifie au moins une séquence cible du matériel nucléique par l'une quelconque des réactions d'amplification enzymatique telles que définies précédemment. Ces techniques mettent généralement une succession de cycle comprenant les étapes suivantes :
o la dénaturation de la séquence cible si celle ci est en double brin afin d'obtenir deux brins cibles, complémentaires,
o l'hybridation de chacun des brins cibles, obtenus lors de l'étape de dénaturation précédente avec au moins une amorce d'amplification,
o la formation à partir des amorces d'amplification des brins complémentaires aux brins sur lesquels elles sont hybridées en présence d'une enzyme polymérase et de nucléosides triphosphate (ribonucléoside triphosphate et/ou desoxyribonucléoside triphosphate selon les techniques)
ce cycle étant répété un nombre de fois déterminé pour obtenir la séquence cible dans une proportion suffisante pour permettre sa détection.

Par hybridation, on entend le processus au cours duquel, dans des conditions appropriées, deux séquences nucléiques telle que notamment une amorce d'amplification et une séquence cible ou une sonde d'hybridation et une séquence cible, se lient avec des liaisons hydrogènes stables et spécifiques pour former un double brin. Ces liaisons hydrogènes se forment entre les bases complémentaires Adénine (A) et thymine (T) (ou uracile (U)) (on parle de liaison A-T) ou entre les bases complémentaires Guanine (G) et cytosine (C) (on parle de liaison GC). L'hybridation de deux séquences nucléiques peut être totale (on parle alors de séquences complémentaires), c'est à dire que le double brin obtenu lors de cette hybridation comprend uniquement des liaisons A-T et des liaisons C-G. Cette hybridation peut être partielle (on parle alors de séquences suffisamment complémentaires), c'est à dire que le double brin obtenu comprend des liaisons A-T et des liaisons C-G permettant de former le double brin, mais également des bases non liées à une base complémentaire. L'hybridation entre deux séquences complémentaires ou suffisamment complémentaires dépend des conditions opératoires qui sont utilisées, et notamment de la stringence. La stringence est définie notamment en fonction de la composition en bases des deux séquences nucléiques, ainsi que par le degré de mésappariement entre ces deux séquences nucléiques. La stringence peut également être fonction des paramètres de la réaction, tels que la concentration et le type d'espèces ioniques présentes dans la solution d'hybridation, la nature et la concentration d'agents dénaturants et/ou la température d'hybridation. Toutes ces données sont bien connues et les conditions appropriées peuvent être déterminées par l'homme du métier. En général, selon la longueur des séquences nucléiques que l'on souhaite hybrider, la température d'hybridation est comprise entre environ 20 et 70°C, en particulier entre 35 et 65°C dans une solution saline à une concentration d'environ 0,5 à 1 M. Lors de l'étape d'hybridation, l'amorce d'amplification selon l'invention s'hybride préférentiellement sur une séquence cible conservée.

Selon un mode préféré de réalisation de l'invention, on amplifie au moins une séquence cible par PCR. Cette réaction d'amplification peut être réalisée par l'hybridation de deux amorces d'amplification qui s'hybrident à chaque extrémité de la séquence cible que l'on souhaite amplifier. Préférentiellement, chaque amorce d'amplification s'hybride sur une séquence cible conservée. A titre indicatif, lorsqu'on utilise une amorce d'amplification comprenant 20 motifs nucléotidiques de la SEQ ID N°1 et une amorce d'amplification comprenant 20 motifs nucléotidiques de la SEQ ID N°2, on amplifie une séquence cible d'une taille de 1375 paires de base. Cette séquence cible correspondrait par exemple à la séquence 128-1503 du génome de *Staphylococcus aureus* (référence Genbank X68417). Cette séquence est alors très pertinente pour l'identification d'espèces de bactéries du genre Staphylococcus puisque une longueur de 1375 paires de bases permet d'obtenir un grand nombre de régions spécifiques de chaque espèce de Staphylocoques, au sein de cette longue séquence, chacune des régions spécifiques pouvant être mise en évidence par l'utilisation d'une batterie de sondes d'espèces.

Selon un mode préféré de réalisation de l'invention, lorsqu'on utilise une amorce d'amplification comprenant 20 motifs nucléotidiques de la SEQ ID N°1 et une amorce d'amplification comprenant 20 motifs nucléotidiques de la SEQ ID N°2 en PCR, chaque cycle de la réaction d'amplification enzymatique comprend les étapes suivantes :
o la dénaturation de la séquence cible si celle ci est en double brin (et/ou la dénaturation de la séquence cible et de l'amplicon à partir du 2^{ème} cycle), réalisée préférentiellement à une température comprise entre 90 et 99°C, et encore plus préférentiellement entre 94 et 95 °C, pour une durée préférentiellement comprise entre 20 et 40 secondes, et encore plus préférentiellement pour une durée comprise entre 25 et 35 secondes
o l'hybridation de chacun des brins cibles, obtenus lors de l'étape de dénaturation précédente avec au moins une amorce d'amplification, réalisée préférentiellement à une température comprise entre 50 et 60 °C, et encore plus préférentiellement entre 53 et 57 °C, pour une durée préférentiellement comprise entre 20 et 40 secondes, et encore plus préférentiellement pour une durée comprise entre 25 et 35 secondes
o la formation à partir des amorces d'amplification de copies de la séquence cible (ou amplicons), réalisée préférentiellement à une température comprise entre 65 et 80°C, et encore plus préférentiellement entre 70 et 74 °C, pour une durée préférentiellement comprise entre 45 et 75 secondes, et encore plus préférentiellement pour une durée comprise entre 55 et 65 secondes
ce cycle étant répété préférentiellement de 25 à 35 fois, et encore plus préférentiellement 30 fois.

Au sens de la présente invention, on entend par amplicons les copies de la séquence cible obtenues lors d'une réaction d'amplification enzymatique.

La détection des amplicons lors de l'étape C, peut être réalisée par tous les protocoles connus de l'homme du métier concernant la détection d'acides nucléiques. A titre indicatif, cette détection peut être réalisée après migration par électrophorèse des amplicons qui migre en fonction de leur taille. Le gel et le milieu de migration peuvent comprendre du bromure d'éthydium afin de permettre la détection directe des amplicons lorsque le gel est placé, après un temps de migration donné, sur une table lumineuse à rayons UV (ultra violet) par l'émission d'un signal lumineux. Ces techniques d'électrophorèse sont bien connus de l'homme du métier. Les amplicons peuvent également être détectés et quantifiés par l'utilisation d'une gamme de quantification obtenue par une réaction d'amplification conduite jusqu'à saturation.

On peut également utiliser des sondes d'hybridation pour détecter la présence des amplicons. Au sens de la présente invention, on entend par sonde d'hybridation, une séquence nucléique de 5 à 100 motifs nucléotidiques, notamment de 15 à 35 motifs nucléotidiques, possédant une spécificité d'hybridation dans des conditions déterminées pour former un complexe d'hybridation avec une séquence nucléique cible. La sonde d'hybridation peut comprendre un marqueur permettant sa détection. On parle alors de sondes de détection. Par détection on entend soit une détection directe par une méthode physique, soit une détection indirecte par une méthode de détection à l'aide d'un marqueur. De nombreuses méthodes de détection existent pour la détection des acides nucléiques. [Voir par exemple Kricka et al., Clinical Chemistry, 1999, n° 45(4), p.453-458 ou Keller G.H. et al., DNA Probes, 2nd Ed., Stockton Press, 1993, sections 5 et 6, p.173-249]. Par marqueur, on entend un traceur capable d'engendrer un signal que l'on peut détecter. Une liste non limitative de ces traceurs comprend les enzymes qui produisent un signal détectable par exemple par colorimétrie, fluorescence ou luminescence, comme la peroxydase de raifort, la phosphatase alcaline, la bêta galactosidase, la glucose-6-phosphate déshydrogénase; les chromophores comme les composés fluorescents, luminescents ou colorants ; les groupements à densité électronique détectables par microscopie électronique ou par leurs propriétés électriques comme la conductivité, par les méthodes d'ampérométrie ou de voltamétrie, ou par des mesures d'impédance ; les groupements détectables par des méthodes optiques comme la diffraction, la résonance plasmon de surface, la variation d'angle de contact ou par des méthodes physiques comme la spectroscopie de force atomique, l'effet tunnel, etc. ; les molécules radioactives comme ³²P, ³⁵S ou ¹²⁵I.

Selon un mode particulier de réalisation de l'invention, le procédé tel que défini ci dessus comprend en outre l'étape suivante :
D. on identifie l'espèce de bactéries de genre *Staphylococcus* par l'utilisation d'au moins une sonde d'hybridation capable de s'hybrider sur une séquence cible spécifique d'une espèce de bactéries de genre Staphylococcus.

Par sonde d'hybridation capable de s'hybrider sur une séquence cible spécifique d'une espèce de bactéries de genre *Staphylococcus*, on entend une sonde d'hybridation telle que définie précédemment, qui comprend au moins une séquence complémentaire ou suffisamment complémentaire à une séquence cible spécifique d'une espèce de bactéries de genre *Staphylococcus.* Au sens de la présente invention, la sonde d'hybridation peut être une sonde dite de détection. Dans ce cas, la sonde dite de détection est marquée au moyen d'un marqueur tel que défini précédemment. Grâce à la présence de ce marqueur, on peut détecter la présence d'une réaction d'hybridation entre une sonde de détection donnée et la séquence cible spécifique d'une espèce donnée. La présence d'une telle réaction d'hybridation permet ainsi d'identifier une espèce donnée de bactéries de genre *Staphylococcus.* La sonde d'hybridation peut être également une sonde dite de capture. Dans ce cas, la sonde dite de capture est immobilisée ou immobilisable sur un support solide par tout moyen approprié, c'est-à-dire directement ou indirectement, par exemple par covalence ou adsorption. On détecte alors la réaction d'hybridation entre une sonde de capture donnée et une séquence cible spécifique de l'espèce donnée ce qui permet d'identifier l'espèce bactérienne. Pour la détection de la réaction d'hybridation, on utilise préférentiellement des séquences cibles marquées, directement (notamment par l'incorporation d'un marqueur au sein de la séquence cible) ou indirectement (notamment par l'utilisation d'une sonde de détection telle que définie précédemment) la séquence cible. On peut notamment réaliser avant l'étape d'hybridation une étape de marquage et/ou de clivage de la séquence cible, par exemple en utilisant un désoxyribonucléotide triphosphate marqué lors de la réaction d'amplification enzymatique. Le clivage peut être réalisé notamment par l'action de l'imidazole et de chlorure de manganèse. La séquence cible peut aussi être marqué après l'étape d'amplification, par exemple en hybridant une sonde de détection selon la technique d'hybridation sandwich décrite dans le document WO 91/19812. Un autre mode particulier préférentiel de marquage d'acides nucléiques est décrit dans la demande FR2 780 059.

Comme support solide, on peut utiliser des matériaux de synthèse ou des matériaux naturels, éventuellement modifiés chimiquement, notamment les polysaccharides tels que les matériaux à base de cellulose, par exemple du papier, des dérivés de cellulose tels que l'acétate de cellulose et la nitrocellulose ou le dextrane, des polymères, des copolymères, notamment à base de monomères du type styrène, des fibres naturelles telles que le coton, et des fibres synthétiques telles que le nylon ; des matériaux minéraux tels que la silice, le quartz, des verres, des céramiques ; des latex ; des particules magnétiques ; des dérivés métalliques, des gels etc. Le support solide peut être sous la forme d'une plaque de microtitration, d'une membrane comme décrit dans la demande WO 94/12670, d'une particule. On peut également immobiliser sur le support plusieurs sondes de capture différentes, chacune étant spécifique d'une espèce de bactéries de genre *Staphylococcus.* Selon un mode préféré de réalisation de l'invention, on utilise comme support une biopuce sur laquelle sont immobilisées un grand nombre de sondes de capture différentes. Par biopuce, on entend un support solide de dimension réduite où sont fixée une multitude de sondes de capture à des positions prédéterminées. Le concept de biopuce, ou puce à ADN, date du début des années 90 et repose sur une technologie pluridisciplinaire intégrant la micro-électronique, la chimie des acides nucléiques, l'analyse d'images et l'informatique. On peut citer à titre indicatif, les puces à ADN mises au point par la société Affymetrix ("Accessing Genetic Information with High-Density DNA arrays", M. Chee et al., Science, 1996, 274, 610-614. "Light-generated oligonucleotide arrays for rapide DNA sequence analysis", A. Caviani Pease et al., Proc. Natl. Acad. Sci. USA, 1994, 91, 5022-5026), pour les diagnostics moléculaires. D'autres exemples de biopuces sont donnés dans les publications de G. Ramsay, Nature Biotechnology, 1998, n°16, p. 40-44 ; F. Ginot, Human Mutation, 1997, n°10, p.1-10 ; J. Cheng et al, Molecular diagnosis, 1996, n°1(3), p.183-200 ; T. Livache et al, Nucleic Acids Research, 1994, n° 22(15), p. 2915-2921 ; J. Cheng et al, Nature Biotechnology, 1998, n° 16, p. 541-546 ou dans les brevets US 4,981,783, US 5,700,637, US 5,445,934, US 5,744,305 et US 5,807,522. La caractéristique principale du support solide doit être de conserver les caractéristiques d'hybridation des sondes de capture sur les séquences cible tout en générant un bruit de fond minimum pour la méthode de détection. La méthode des biopuces repose sur l'emploi de sondes de capture fixées sur un support solide sur lesquelles on fait agir des séquences nucléiques cibles marqués directement ou indirectement avec par exemple des fluorochromes. Les sondes de capture sont positionnées de manière spécifique sur le support ou puce et chaque hybridation donne une information particulière, en relation avec la séquence nucléique cible. Après hybridation, le support ou puce est lavé(e), et la réaction d'hybridation entre une séquence nucléique cible marquée et une sonde de capture peut être détecté par l'utilisation d'un ligand de forte affinité lié par exemple à un marqueur de type fluorochrome. La fluorescence est lue par exemple par un scanner et l'analyse de la fluorescence est traitée par informatique. La présence d'une réaction d'hybridation entre une sonde de capture donnée et une séquence cible spécifique d'une espèce donnée permet ainsi d'identifier l'espèce donnée de bactéries de genre *Staphylococcus.*

L'invention concerne également une amorce d'amplification caractérisée en ce qu'elle comprend au moins 10, préférentiellement au moins 15 et encore plus préférentiellement au moins 20 motifs nucléotidiques de la SEQ ID N°1 et une amorce d'amplification caractérisée en ce qu'elle comprend au moins 10, préférentiellement au moins 15 et encore plus préférentiellement au moins 20 motifs nucléotidiques de la SEQ ID N°2.

Préférentiellement, l'amorce d'amplification selon l'invention comprend au moins 10, préférentiellement au moins 15 en encore plus préférentiellement au moins 20 motifs nucléotidiques contigus de la SEQ ID N°1 ou au moins 10, préférentiellement au moins 15 en encore plus préférentiellement au moins 20 motifs nucléotidiques contigus de la SEQ ID N°2.

Au sens de la présente invention, une amorce d'amplification comprenant
- une séquence homologue à la SEQ ID N°1 ou la SEQ ID N°2, c'est à dire
   o la séquence complémentaire ou suffisamment complémentaire de la SEQ ID N°1 ou 2
   o une séquence présentant 80 % d'homologie avec la SEQ ID N°1 ou la SEQ ID N°2,
- une séquence comprenant une séquence de SEQ ID N°1 ou SEQ ID N°2 (ou une séquence homologue à SEQ ID N°1 ou SEQ ID N°2 telle que définie précédemment) dans laquelle les bases uracile sont substituées aux bases thymine, ou dans laquelle au moins une base est substituée par une base universelle telle que l'inosine,
et qui aurait la même fonction que l'amorce d'amplification selon l'invention, c'est à dire permettre l'amplification d'un grand nombre d'espèces de bactéries de genre *Staphylococcus,* est considérée comme équivalente à l'amorce d'amplification selon l'invention.

L'invention concerne également une paire d'amorces d'amplification caractérisé en ce qu'elle comprend au moins une amorce d'amplification de 15 à 25 motifs nucléotidiques comprenant au moins 10 motifs nucléotidiques de la SEQ ID N°1 et au moins une amorce d'amplification de 15 à 25 motifs nucléotidiques comprenant au moins 10 motifs nucléotidiques de la SEQ ID N°2.

Cette paire d'amorce est préférentiellement utilisée dans une réaction d'amplification enzymatique telle que la PCR.

L'utilisation d'au moins une amorce comprenant au moins 10, préférentiellement au moins 15 et encore plus préférentiellement au moins 20 motifs nucléotidiques de la SEQ ID N°1 et/ou au moins une amorce comprenant au moins 10, préférentiellement au moins 15 et encore plus préférentiellement au moins 20 motifs nucléotidiques de la SEQ ID N°2 pour la détection et/ou l'identification de bactéries appartenant au genre *Staphylococcus* est décrite.

Un kit de diagnostic de bactéries de genre *Staphylococcus* comprenant au moins une amorce comprenant au moins 10, préférentiellement au moins 15 et encore plus préférentiellement au moins 20 motifs nucléotidiques de la SEQ ID N°1 et/ou au moins une amorce comprenant au moins 10, préférentiellement au moins 15 et encore plus préférentiellement au moins 20 motifs nucléotidiques de la SEQ ID N°2, est également décrit.

Sont également décrites une sonde d'hybridation caractérisée en ce qu'elle comprend au moins 10, préférentiellement au moins 15 et encore plus préférentiellement au moins 20 motifs nucléotidiques de la SEQ ID N°1 et une sonde d'hybridation caractérisée en ce qu'elle comprend au moins 10, préférentiellement au moins 15 et encore plus préférentiellement au moins 20 motifs nucléotidiques de la SEQ ID N°2.

Préférentiellement, la sonde d'hybridation comprend au moins 10, préférentiellement au moins 15 en encore plus préférentiellement au moins 20 motifs nucléotidiques contigus de la SEQ ID N°1 ou au moins 10, préférentiellement au moins 15 en encore plus préférentiellement au moins 20 motifs nucléotidiques contigus de la SEQ ID N°2.

Une sonde d'hybridation comprenant
- une séquence homologue à la SEQ ID N°1 ou la SEQ ID N°2, c'est à dire
   o la séquence complémentaire ou suffisamment complémentaire de la SEQ ID N°1 ou 2
   o une séquence présentant 80 % d'homologie avec la SEQ ID N°1 ou la SEQ ID N°2,
- une séquence comprenant une séquence de SEQ ID N°1 ou SEQ ID N°2 (ou une séquence homologue à SEQ ID N°1 ou SEQ ID N°2 telle que définie précédemment) dans laquelle les bases uracile sont substituées aux bases thymine, ou dans laquelle au moins une base est substituée par une base universelle telle que l'inosine,
et qui aurait la même fonction que la sonde d'hybridation décrite, c'est à dire permettre la détection d'un grand nombre d'espèces de bactéries de genre *Staphylococcus,* est considérée comme équivalente à la sonde d'hybridation décrite.

La sonde d'hybridation décrite peut être une sonde de détection ou une sonde de capture telles que définies précédemment.

Est également décrite l'utilisation d'au moins une sonde d'hydridation comprenant au moins 10, préférentiellement au moins 15 et encore plus préférentiellement au moins 20 motifs nucléotidiques de la SEQ ID N° 1 et/ou au moins une sonde d'hybridation comprenant au moins 10, préférentiellement au moins 15 et encore plus préférentiellement au moins 20 motifs nucléotidiques de la SEQ ID N°2 pour la détection et/ou l'identification de bactéries appartenant au genre *Staphylococcus.*

Un kit de diagnostic de bactéries de genre *Staphylococcus* comprenant au moins une sonde d'hybridation comprenant au moins 10, préférentiellement au moins 15 et encore plus préférentiellement au moins 20 motifs nucléotidiques de la SEQ ID N°1 et/ou au moins une sonde d'hybridation comprenant au moins 10, préférentiellement au moins 15 et encore plus préférentiellement au moins 20 motifs nucléotidiques de la SEQ ID N°2, est également décrit.

Enfin une composition pour la détection de bactéries de genre *Staphylococcus* comprenant au moins une sonde d'hybridation comprenant au moins 10, préférentiellement au moins 15 et encore plus préférentiellement au moins 20 motifs nucléotidiques de la SEQ ID N°1 et/ou au moins une sonde d'hybridation comprenant au moins 10, préférentiellement au moins 15 et encore plus préférentiellement au moins 20 motifs nucléotidiques de la SEQ ID N°2, est décrite.

La figure suivante est donnée à titre illustratif et n'a aucun caractère limitatif. Elle permettra de mieux comprendre l'invention.

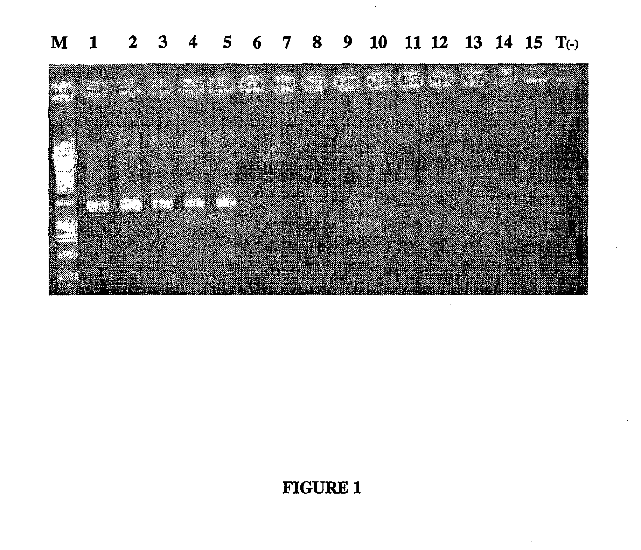

La figure 1 présente une photographie d'un gel d'électrophorèse tel qu'obtenu après migration de différents amplicons obtenus selon l'exemple ci après, déposés dans les puits 1 à 15. Les amplicons provenaient de différentes espèces de bactéries de genre Staphylococcus (puits 1 : Puits 1: *Staphylococcus cohnii* ssp cohnii; Puits 2: *Staphylococcus cohnii* ssp urealyticum: Puits 3: *Staphylococcus intermedius;* Puits 4 : *Staphylococcus xylosus;* Puits 5 : *Staphylococcus pasteuri*) et d'espèces de bactéries n'appartenant pas au genre Staphylococcus (Puits 6: *Streptococcus pneumoniae;* Puits 7: *Klebsiella pneumoniae;* Puits 8: *Pseudomonas aeruginosa;* Puits 9 *Enterococcus faecalis;* Puits 10: *Enterobacter cloacae;* Puits 11: *Proteus mirabilis;* Puits 12: *Streptococcus agalactiae;* Puits 13: *Citrobacter freundii;* Puits 14 : *Salmonella enteritidis;* Puits 15: *Stenotrophomonas maltophila*). La bonne taille des amplicons a été vérifiée par la migration simultanée d'un marqueur de taille (puit M). La présence de bactérie de genre *Staphylococcus* était confirmée par la présence d'une bande de la taille attendue, correspondant à un amplicon de 1,4kB environ (puits 1 à 5), alors qu'aucune bande n'était observée pour les bactéries n'appartenant pas au genre *Staphylococcus* (puits 6 à 15). Le puits T(-) correspond à un tube témoin négatif.

Les exemples suivants sont donnés à titre illustratif et n'ont aucun caractère limitatif. Ils permettront de mieux comprendre l'invention.*
*1*/*Mise en culture de bactéries:* 31 espèces de bactéries de genre *Staphylococcus* ainsi que 11 espèces de bactéries n'appartenant pas au genre *Staphylococcus* (tableau 1) ont été mise en culture sur milieu gélosé trypcase-soja par une incubation à 37°C durant une nuit. Le n° de collection des espèces de bactéries mises en culture est indiqué dans le tableau 1 ci après (ATCC : American type culture collection ; DSM : Deutsche Sammlung von Mikroorganismen; CCM : Czech collection of microorganisms; CCUG : Culture collection, université de Göteborg, Suède).

**Tableau 1- Genre et espèce de bactéries testées dans le procédé selon l'invention**

| **Genre** | **Espèce** | **n° collection** |
|---|---|---|
| *Staphylococcus* | *arlettae* | ATCC43957 |
| *Staphylococcus* | *aureus.aureus* | ATCC 25923 |
| *Staphylococcus* | *aureus.aureus* | ATCC35844T |
| *Staphylococcus* | *auricularis* | ATCC33753T |
| *Staphylococcus* | *capitis.capitis* | ATCC27840T |
| *Staphylococcus* | *capitis.ureolyticus* | ATCC49325 |
| *Staphylococcus* | *caprae* | ATCC35538T |
| *Staphylococcus* | *carnosus carnosus* | ATCC51365T |
| *Staphylococcus* | *carnosus utilis* | DSM11676T |
| *Staphylococcus* | *chromogenes* | ATCC43764T |
| *Staphylococcus* | *cohnii cohnii* | ATCC29974T |
| *Staphylococcus* | *cohnii urealyticum* | ATCC49330T |
| *Staphylococcus* | *condimenti* | DSM11674T |
| *Staphylococcus* | *delphini* | ATCC49171T |
| *Staphylococcus* | *epidermidis* | ATCC14990T |
| *Staphylococcus* | *equorum* | ATCC43958T |
| *Staphylococcus* | *gallinarum* | ATCC35539T |
| *Staphylococcus* | *haemolyticus* | ATCC29970T |
| *Staphylococcus* | *hominis.hominis* | ATCC27844T |
| *Staphylococcus* | *hominis.novobiosepticus* | CCUG42399T |
| *Staphylococcus* | *hyicus* | ATCC11249T |
| *Staphylococcus* | *intermedius* | ATCC29663T |
| *Staphylococcus* | *kloosii* | ATCC43959T |
| *Staphylococcus* | *lentus* | ATCC29070T |
| *Staphylococcus* | *lugdunensis* | ATCC43809T |
| *Staphylococcus* | *pasteuri* | ATCC51129T |
| *Staphylococcus* | *piscifermentans* | ATCC51136T |
| *Staphylococcus* | *pulvereri* | ATCC51698T |
| *Staphylococcus* | *saprophyticus.bovis* | CCM4410T |
| *Staphylococcus* | *saprophyticus.saprophyticus* | ATCC15305T |
| *Staphylococcus* | *schleiferi.coagulans* | CCUG37248T |
| *Staphylococcus* | *schleiferi.schleiferi* | ATCC4308T |
| *Staphylococcus* | *sciuri* | ATCC 29062T |
| *Staphylococcus* | *simulans* | ATCC27848T |
| *Staphylococcus* | *virulinus* | - |
| *Staphylococcus* | *warneri* * | ATCC27836T |
| *Staphylococcu*s | *xylosus* | ATCC29971T |
| *Escherichia* | *coli* | ATCC11775T |
| *Streptococcus* | *pneumoniae* | ATCC33400T |
| *Klebsiella* | *pneumoniae* | ATCC13883T |
| *Pseudomonas* | *aeruginosa* | ATCC10145T |
| *Enterococcus* | *faecalis* | ATCC19433T |
| *Enterobacter* | *cloacae* | ATCC13047T |
| *Proteus* | *mirabilis* | ATCC29906T |
| *Streptococcus* | *agalactiae* | ATCC13813T |
| *Citrobacter* | *freundii* | ATCC8090T |
| *Salmonella* | *enteritidis* | ATCC13076 |
| *Stenotrophomonas* | *maltophila* | ATCC13637T |

Une suspension homogène à environ 10⁸ CFU/ml dans de l'eau stérile a été préparée à partir des différentes colonies de bactéries cultivées, par un densitomètre bioMérieux (ref 99234). Les bactéries contenues dans un volume de 600 µl de cette suspension ont été lysées mécaniquement afin d'extraire leur matériel nucléique. L'amplification a été réalisée sur ce lysat de bactéries.
*2) Amplification d'une séquence cible de bactéries de genre Staphylococcus par Polymérase Chain Réaction (PCR)*: l'amplification d'une séquence cible a été réalisée par PCR pour chaque souche bactérienne, par l'utilisation d'un kit Expand high fidelity de Roche (réf 11732650). Les PCR ont été effectuées à l'aide du kit Fast-Start^{™} (Roche Molecular Biochemicals). Chaque PCR a été effectuée dans un volume final de 50 µl contenant du MgCl2 (0,25mM), des deoxynucleotides triphosphate (0,25 mM Roche), 0,3 µM de l'amorce d'amplification comprenant 20 motifs nucléotidiques de la SEQ ID N°1 (5' TAACCTACCTATAAGACTGG 3') et 0,3 µM de l'amorce d'amplification comprenant 20 motifs nucléotidiques de la SEQ ID N°2 (5' TC ACC CCA ATC ATT TGT CCC 3') et 0,05 U du mélange de polymérases Expand high fidelity du kit afin d'obtenir un volume réactionnel final de 50µL.
Après 4 min à 95°C, 30 cycles de PCR ont été réalisés. Chaque cycle comprenait une étape de dénaturation (30 s à 95°C) ; une étape d'hybridation des amorces d'amplification sur la séquence cible (30 s à 55°C) ; une étape de formation des amplicons (1 min à 72°C). Une extension finale de 7 min à 72°C terminait la réaction d'amplification enzymatique.
*3) Vérification de l'amplification:* La qualité de l'amplification a été vérifiée sur gel 1,5% d'agarose dans du tampon TBE 0,5X, après coloration au bromure d'ethidium. La bonne taille des amplicons a été vérifiée par la migration simultanée d'un marqueur de taille (Smart Ladder, Eurogentec). La présence de bactérie de genre *Staphylococcus* était confirmée par la présence d'une bande de la taille attendue, correspondant à un amplicon de 1,4kB environ, alors qu'aucune bande n'était observée pour les bactéries n'appartenant pas au genre Staphylococcus.
*4) Sensibilité des amorces d'amplification selon l'invention* : la sensibilité des amorces a été testée sur de l'ADN génomique. Pour cela, une gamme d'ADN génomique (2.10⁷; 2.10⁶ ; 2.10⁵ ; 2.10⁴ ; 2.10³ ; 2.10² et 2.10¹ copies / µl) été réalisée pour sensibilité des amorces et testé en PCR selon un protocole comparable à celui décrit dans le paragraphe 2) de l'exemple. Des amplicons étaient obtenus pour des concentrations d'ADN génomique supérieures ou égales à 2.10⁵ copies/µl.
*5) Spécificité des amorces :* un amplicon d'environ 1400 paires de bases a été obtenu pour chacune des 39 espèces de bactéries de genre *Staphylococcus* alors qu'aucun amplicon n'a été généré pour les autres espèces de bactéries n'appartenant pas au genre *Staphylococcus* montrant ainsi la très bonne spécificité des amorces selon l'invention, et plus particulièrement de la paire d'amorces selon l'invention. A titre indicatif, un exemple de gel d'électrophorèse obtenue après migration de différents amplicons provenant de différentes espèces de bactéries de genre Staphylococcus (*Staphylococcus cohnii* ssp cohnii; *Staphylococcus cohnii* ssp urealyticum ; *Staphylococcus intermedius; Staphylococcus xylosus; Staphylococcus pasteuri*) et d'espèces de bactéries n'appartenant pas au genre Staphylococcus (*Streptococcus pneumoniae; Klebsiella pneumoniae; Pseudomonas aeruginosa;:Enterococcus faecalis; Enterobacter cloacae; Proteus mirabilis; Streptococcus agalactiae; Citrobacter freundii; Salmonella enteritidis; Stenotrophomonas maltophila*) est présenté figure 1.

Le rendement d'amplification a été évaluée sur un bioanalyser 2100 d'Agilent. On obtenait un bon rendement d'amplification d'environ 18 ng / µl

Des résultats comparables étaient obtenus à partir de prélèvements d'échantillons cliniques (Hôpital Universitaire de Genève) comprenant les bactéries suivantes : *S*. *arlettae ; S. auricularis; S. aureus; S. capitis; S. caprae ; S. carnosus; S. caseolyticus ; S. chromogenes ; S. cohnii; S. condimenti ; S. delphini ; S. epidermidis ; S. equorum ; S. felis ; S. gallinarum; S. haemolyticus ; S. hominis ; S. hyicus ; S. intermedius ; S. kloosil ; S. lentus ; S. lugdunensis ; S. muscae ;S. pasteuri ; S. piscifermentans ; S. pulvereti*/ *S. vitulinus ; S. saccharolyticus ; S. saprophyticus ; S. schleiferi ; S. sciuri ; S. simulans ; S. warneri ; S.xylosus*

## Revendications

1. Procédé de détection et/ou d'identification de bactéries de genre *Staphylococcus* d'un échantillon biologique comprenant les étapes suivantes :
A. on extrait le matériel nucléique des bactéries de genre *Staphylococcus,*
B. on amplifie au moins une séquence cible du matériel nucléique des bactéries de genre *Staphylococcus* grâce à au moins une amorce d'amplification de 15 à 25 motifs nucléotidiques comprenant au moins 10 motifs nucléotidiques de la SEQ ID N°1 et au moins une amorce d'amplification de 15 à 25 motifs nucléotidiques comprenant au moins 10 motifs nucléotidiques de la SEQ ID N°2 pour obtenir des amplicons de la séquence cible
C. on détermine la présence de bactéries de genre *Staphylococcus* par la détection desdits amplicons.

2. Procédé de détection et/ou d'identification de bactéries appartenant au genre *Staphylococcus* selon la revendication 1 comprenant en outre l'étape suivante :
D. on identifie l'espèce de bactéries de genre *Staphylococcus* par l'utilisation d'au moins une sonde d'hybridation capable de s'hybrider sur une séquence cible spécifique d'une espèce de bactérie de genre *Staphylococcus.*

3. Paire d'amorces d'amplification **caractérisé en ce qu'**elle comprend au moins une amorce d'amplification de 15 à 25 motifs nucléotidiques comprenant au moins 10 motifs nucléotidiques de la SEQ ID N° 1 et au moins une amorce d'amplification de 15 à 25 motifs nucléotidiques comprenant au moins 10 motifs nucléotidiques de la SEQ ID N°2.

4. Utilisation d'au moins une paire d'amorce telle que définie dans la revendication 3 pour la détection et/ou l'identification de bactéries de genre *Staphylococcus:*

5. Kit de diagnostic de bactéries de genre *Staphylococcus* comprenant au moins une paire d'amorce telle que définie dans la revendication 3.

## Claims

1. A method for detecting and/or identifying bacteria of the genus *Staphylococcus* in a biological sample, comprising the following steps:
a) the nucleic acid material of the bacteria of the genus *Staphylococcus* is extracted,
b) at least one target sequence of the nucleic acid material of the bacteria of the genus *Staphylococcus* is amplified using at least one amplification primer, which comprises from 15 to 25 nucleotide motifs, comprising at least 10 nucleotide motifs of SEQ ID No. 1 and at least one amplification primer, which comprises from 15 to 25 nucleotide motifs, comprising at least 10 nucleotide motifs of SEQ ID No. 2, in order to obtain amplicons of the target sequence,
c) the presence of bacteria of the genus *Staphylococcus* is determined by detecting said amplicons.

2. The method for detecting and/or identifying bacteria belonging to the genus *Staphylococcus* as claimed in claim 1, additionally comprising the following step:
d) the bacterial species belonging to the genus *Staphylococcus* is identified by using at least one hybridization probe which is able to hybridize with a target sequence which is specific for a bacterial species belonging to the genus *Staphylococcus.*

3. A pair of amplification primers, **characterized in that** it comprises at least one amplification primer, which comprises from 15 to 25 nucleotide motifs, comprising at least 10 nucleotide motifs of SEQ ID No. 1 and at least one amplification primer, which comprises from 15 to 25 nucleotide motifs, comprising at least 10 nucleotide motifs of SEQ ID No. 2.

4. The use of at least one pair of amplification primers as defined in claim 3 for detecting and/or identifying bacteria of the genus *Staphylococcus.*

5. A kit for diagnosing bacteria of the genus *Staphylococcus,* comprising at least one pair of amplification primers as defined in claim 3.

## Patentansprüche

1. Verfahren zum Nachweisen und/oder Identifizieren von Bakterien der Gattung *Staphylococcus* von einer biologischen Probe, das die folgenden Schritte umfaßt:
A. Man extrahiert das Nukleinsäurematerial der Bakterien der Gattung *Staphylococcus,*
B. Man amplifiziert mindestens eine Zielsequenz des Nukleinsäurematerials der Bakterien der Gattung *Staphylococcus* mittels mindestens einem Amplifikationsprimer mit 15 bis 25 Nukleotidmotiven, umfassend mindestens 10 Nukleotidmotive von SEQ ID NR. 1 und mindestens einem Amplifikationsprimer mit 15 bis 25 Nukleotidmotiven, umfassend mindestens 10 Nukleotidmotive von SEQ ID Nr. 2, um zu den Amplikons der Zielsequenz zu gelangen,
C. Man bestimmt das Vorliegen von Bakterien der Gattung *Staphylococcus* durch Nachweisen der Amplikons.

2. Verfahren zum Nachweisen und/oder Identifizieren von Bakterien der Gattung *Staphylococcus* nach Anspruch 1, das weiterhin den folgenden Schritt umfaßt:
D. Man identifiziert die Bakterienart der Gattung *Staphylococcus* durch Einsetzen von mindestens einer Hybridisierungssonde, die fähig ist, mit einer für eine Bakterienart der Gattung *Staphylococcus* spezifischen Zielsequenz zu hybridisieren.

3. Amplifikationsprimerpaar, **dadurch gekennzeichnet, daß** es mindestens einen Amplifikationsprimer mit 15 bis 25 Nukleotidmotiven, umfassend mindestens 10 Nukleotidmotive von SEQ ID NR. 1, und mindestens einen Amplifikationsprimer mit 15-25 Nukleotidmotiven, umfassend mindestens 10 Nukleotidmotive für SEQ ID NR. 2, umfaßt.

4. Verwendung von mindestens einem Primerpaar gemäß Anspruch 3 zum Nachweisen und/oder Identifizieren von Bakterien der Gattung *Staphylococcus.*

5. Kit für die Diagnose von Bakterien der Gattung *Staphylococcus,* das mindestens ein Primerpaar gemäß Anspruch 3 umfaßt.
